# EUROPÄISCHE PATENTSCHRIFT

(11) **EP 1 443 853 B1**
(45) Veröffentlichungstag und Bekanntmachung des Hinweises auf die Patenterteilung: **14.09.2005**
(21) Anmeldenummer: 02785381.1
(22) Anmeldetag: 11.11.2002
(51) Int. Cl.: A61B 3/135

(54) **BELEUCHTUNGSEINHEIT ZUR ERZEUGUNG VON OPTISCHEN SCHNITTBILDERN IN TRANSPARENTEN MEDIEN, INSBESONDERE IM AUGE**
ILLUMINATION UNIT FOR THE GENERATION OF OPTICAL SECTIONAL IMAGES IN TRANSPARENT MEDIA IN PARTICULAR IN THE EYE
UNITE D'ECLAIRAGE PERMETTANT DE PRODUIRE DES VUES EN COUPE OPTIQUES DANS DES MILIEUX TRANSPARENTS, EN PARTICULIER DANS L'OEIL

(30) Priorität: 12.11.2001 DE 10155464
(43) Veröffentlichungstag der Anmeldung: 11.08.2004
(73) Patentinhaber: Carl Zeiss Meditec AG, 07745 Jena (DE)
(72) Erfinder: KOSCHMIEDER, Ingo, 07743 Jena (DE)
(74) Vertreter: Muhsfeldt, Willi
(86) Internationale Anmeldenummer: PCT/EP2002/012561
(87) Internationale Veröffentlichungsnummer: WO 2003/041573

(56) Entgegenhaltungen:
- EP-A- 0 380 197
- US-A- 4 213 678
- US-A- 4 877 321
- US-A- 5 784 146
- PATENT ABSTRACTS OF JAPAN vol. 014, no. 063 (C-0685), 6. Februar 1990 (1990-02-06) & JP 01 285242 A (NIDEK CO LTD), 16. November 1989 (1989-11-16)

## Beschreibung

Die vorliegende Erfindung betrifft eine spezielle Beleuchtungseinheit zur Erzeugung einer veränderbaren Spaltabbildung wie sie in ophthalmologischen Untersuchungsgeräten, u.a. in Spaltlampen, eingesetzt werden. Mittels Spaltbildprojektion wird in dem zu untersuchenden mehr oder weniger transparenten Objekt, z. B. im Inneren des Auges, ein Lichtschnitt erzeugt. Die Parameter des Schnittbündels sind variabel, insbesondere der Einfallswinkel, die Abmessungen des Spaltbildes, seine Intensität und die spektrale Zusammensetzung. Aus der Form, Lage und Intensität des Streulichtes des so erzeugten Schnittbildes können Rückschlüsse auf das zu untersuchende Objekt gewonnen werden.

Bei Spaltlampen wie sie beispielsweise in [1] beschrieben sind, werden zur Erzeugung von Spaltabbildungen bisher mechanisch/optische Elemente, wie Spaltblenden benutzt. Die für eine hohe optische Detailauflösung innerhalb des optischen Schnittes erforderlichen variablen und möglichst geringen Spaltbreiten sind sehr schwer realisierbar. Außerdem sind die mechanischen Baugruppen sehr aufwendig zu justieren, was unter anderem durch die Wärmeausdehnung der Baugruppen noch erschwert wird. Eine Reproduzierbarkeit von Einstellungen zu Messzwecken ist nur beschränkt möglich. Durch die festen Spalt- und Lochblenden und deren Platzbedarf ist die Vielfalt denkbarer Leuchtfeldgeometrien äußerst begrenzt.

Da es sich bei der Spaltbildprojektion um eine optische Abbildung mit physikalisch begrenzter Schärfentiefe handelt, muss die Abbildung immer streng auf den Ort der Untersuchung fokussiert werden. Ein in der gesamten Ausdehnung des menschlichen Auges scharfes Schnittbündel lässt sich mit den bisher genannten Lösungen nicht erzielen. Eine Verbesserung der Verhältnisse kann zwar mit einer Anordnung nach dem Scheimpflugprinzip erreicht werden, jedoch ist der technische Aufwand dafür entsprechend größer.

In der DE 198 12 050 A1 sind ein Verfahren und eine Anordnung zur Beleuchtung bei einem Augenmikroskop beschrieben. Die verschiedensten Leuchtmarkengeometrien werden mit Hilfe opto-elektronischer Bauelemente erzeugt. Die Leuchtfeldgeometrien werden dabei auf den Augenvorder- oder Hintergrund projiziert und dienen der allgemeinen Untersuchung des Auges.

In den Patentschriften US 5,404,884; US 5,139,022 und US 6,275,718 sind Verfahren und Anordnungen zur Beleuchtung der vorderen Augensegmente beschrieben, bei denen als Lichtquelle ein planar konfigurierter Laser verwendet wird. Nachteilig bei diesen Lösungen sind jedoch unter Umständen die eingeschränkte Variabilität der Spaltabmessungen, die verwendete Wellenlänge der Laserquellen und die fehlende Möglichkeit zur Erzeugung von Mehrfachspaltprojektionen. Die beschriebenen Anordnungen sind keine Spaltlampengeräte, die im normalen Diagnosebetrieb genutzt werden. Weiterhin hat das Aufnahmesystem für das Streulicht vom Auge eine physikalisch begrenzte Schärfentiefe, die den Ausdehnungsbereich des scharfen Laser-Schnittbildes nicht vollständig erfassen kann.

Ein Spaltlampenmikroskop zur Untersuchung des Fundus, der Linse und anderer Gewebestrukturen eines Auges wird in der US 4,877,321 beschrieben. Die beschriebene Anordnung basiert auf insgesamt vier Spiegeln, wobei zur Spalterzeugung zwei getrennt bewegliche Scannerspiegel notwendig sind. Die beschriebene Anordnung verfügt weiterhin über Verstellmittel für x und y, mit denen der Spalt in Länge und Breite verändert werden kann. Die erzeugte Abbildung bleibt in jedem Fall ein klassischer Spalt. Um für das jeweilige Projektionsbild eine möglichst gleiche Intensität zu gewährleisten, kann die Intensitätsverstellung an die erzeugte Spaltbreite angepasst werden.

Nachteilig wirkt sich bei der beschriebenen Lösung aus, dass nur ein einfaches Spaltbild darstellbar ist, welches in Länge und/oder Breite variiert werden kann. Durch die Mehr-Spiegelanordnung ist der Gesamtaufbau sehr umfangreich und schränkt die Anwendbarkeit wesentlich ein.

### Literatur:

[1] Rassow, B. u. a., "Ophthalmologisch-optische Instrumente", 1987, Ferdinand Enke Verlag Stuttgart, S. 99 ff und 137 ff

Der vorliegenden Erfindung liegt die Aufgabe zugrunde, die bekannten Lösungen dahingehend weiter zu entwickeln, dass sich neben der klassischen Spaltabbildung beliebige aber definierte Strukturen erzeugen lassen, die mit einer hohen optischen Detailauflösung und einer großen Schärfentiefe in das zu untersuchende Auge projiziert und mit analoger oder digitaler Bildaufnahmetechnik; ausgewertet werden können. Die Lösung soll dabei für eine möglichst breite Anwendung einen einfachen und kompakten Aufbau aufweisen.

Erfindungsgemäß wird die Aufgabe durch die Merkmale der unabhängigen Ansprüche gelöst. Bevorzugte Weiterbildungen und Ausgestaltungen sind Gegenstand der abhängigen Ansprüche.

Mit der vorgeschlagenen technischen Lösung einer Beleuchtungseinheit zur Erzeugung von optischen Schnittbildern im Auge wird durch die gezielte periodische Strahlablenkung eines besonders feinen Laserstrahls mit hoher Schärfentiefe ein Schnittbild erzeugt, welches in der gesamten Dimension des zu untersuchenden Objektes scharf bleibt und eine verbesserte Auswertung ermöglicht. Die Intensität des Laserstrahlbündels kann dabei so variiert werden, das sie für die Beobachtung und Dokumentation ausreicht, der Durchmesser des Strahlenbündels jedoch fein genug für eine hohe Detailauflösung ist. Die vorgeschlagene Beleuchtungseinheit könnte dabei so ausgestaltet werden, dass sie zusätzlich zu einer bereits vorhandenen Beleuchtungseinheit eines ophthalmologischen Gerätes, wie beispielsweise einer Spaltlampe, genutzt wird. Als ein Zusatzmodul für bereits vorhanden ophthalmologische Geräte könnte es durch eine breite Anwendung die Untersuchungen am menschlichen Auge wesentlich vereinfachen und die Genauigkeit der Untersuchungsergebnisse verbessern.

Die Erfindung wird nachfolgend anhand eines Ausführungsbeispieles beschrieben. Dazu zeigen
- Figur 1:: die schematische Darstellung einer Spaltlampe mit dererfindungsgemäßen Beleuchtungseinheit und
- Figur 2:: die Prinzipdarstellung eines Mikroscannerspiegels.

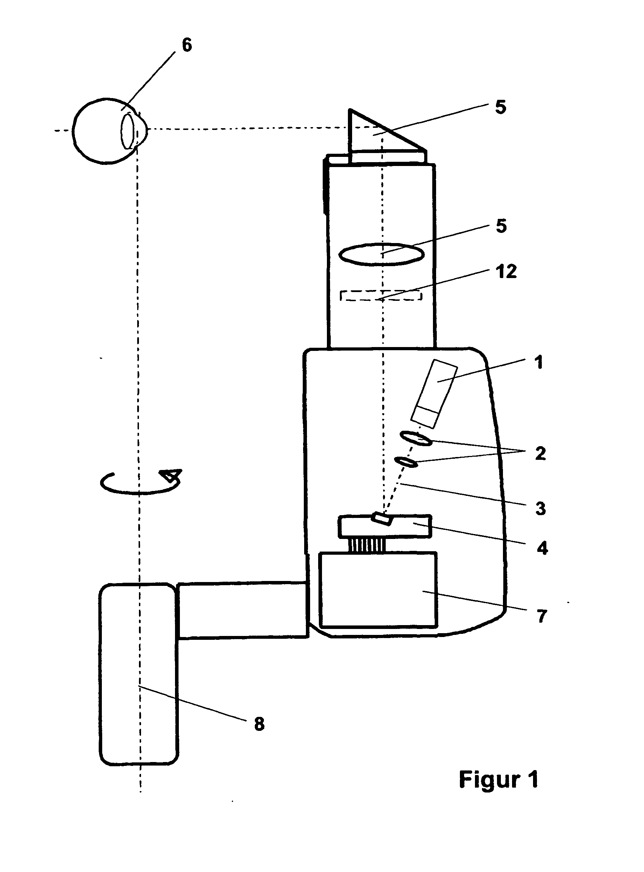

**Figur 1** zeigt den Aufbau einer Spaltlampe mit der erfindungsgemäßen Beleuchtungseinheit zur Erzeugung von optischen Schnittbildern. Die von dem, als Beleuchtungsquelle **1** dienende, Laser ausgehenden Strahlen geringer Divergenz werden durch ein definiert steuerbares Reflexionselement **4** und im Strahlengang vorhandene Strahlformungs- und Strahllenkungselemente (schematisch dargestellt) **5** auf oder in dem zu untersuchenden Auge **6** abgebildet. Dabei sind der Winkel und die Richtung des auf das zu untersuchende Auge gerichteten Laserstrahles **3** frei wählbar.

Als Beleuchtungsquelle **1** wird beispielsweise ein Laserdiodenmodul, vorzugsweise im blauen oder grünen Spektralbereich, eingesetzt. Durch die Beleuchtung mit Licht im grünen oder blauen Spektralbereich erfolgt eine höhere Streuung in den transparenten Medien des Auges, was zu deutlicheren Schnittbildern für die optische Diagnose führt. Mit Hilfe einer Fokussieroptik **2** wird ein sehr feiner Laserstrahl **3** geringer Divergenz erzeugt. Dieser Laserstrahl **3** trifft unter einem bestimmten Winkel und Abstand auf das Reflexionselement **4,** welches in Frequenz und Amplitude von der Steuereinheit **7** steuerbar ist. Das Reflexionselement **4** ist dabei ein Spiegel geringer Abmessungen, der in zwei oder mehreren Richtungen unabhängig voneinander kippbar ist. Dieser sogenannte Mikroscannerspiegel-Chip gehört vorzugsweise zur Gruppe der MEMS (micro-electro-mechanical-system) und kann beliebiger Bauart sein. Vom Fraunhofer-Institut für Mikroelektronische Schaltungen und Systeme Dresden wird beispielsweise ein derartiger Chip unter der Bezeichnung "Resonanter 1 D- und 2D-Mikroscannerspiegel" angeboten.

Als Reflexionselement kann dabei auch ein Mikroscannerspiegel-Chip vom DMD-Typ (digital mirror device) eingesetzt werden, wie er beispielsweise von Texas Instruments angeboten wird.

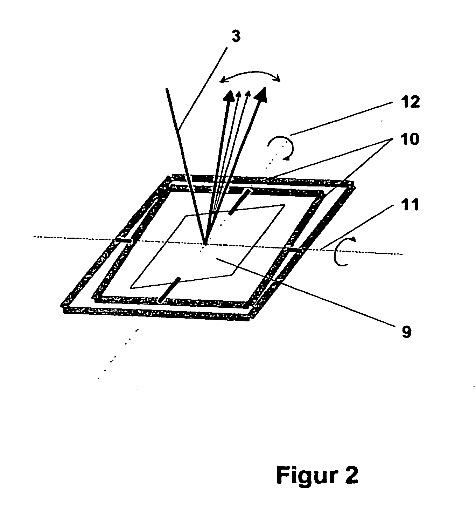

**Figur 2** zeigt die Prinzipdarstellung eines Mikroscannerspiegel-Chips vom MEMS-Typ. Dabei ist das eigentliche Spiegelelement **9** über die Halterahmen **10** entlang der Achsen **11** und **12** kippbar. Die Verkippung um die jeweiligen Achsen **11** und **12** kann beispielsweise durch die Erzeugung von harmonischen mechanischen Schwingungen geeigneter Frequenz und Amplitude mittels elektrostatischer Ansteuerung erfolgen. Der über Torsionsfedem oder kardanische Aufhängungen befestigte Mikroscannerspiegel wird dadurch in harmonische mechanische Schwingungen versetzt. Somit können aus dem punktförmigen Laserstrahl **3** geringer Divergenz Abbildungen der verschiedensten Formen auf oder in dem zu untersuchenden Auge erzeugt werden. Schwingt der Mikroscannerspiegel beispielsweise nur in einer Richtung, so wird das Abbild eines Spaltes erzeugt.

Bei den Mikroscannerspiegel-Chips des Fraunhofer-Institutes liegen die einstellbaren Schwingungsfrequenzen in Abhängigkeit von der darzustellenden Abbildung zwischen 150Hz und 20kHz. Die Amplitude der Schwingung ist bei diesem Mikroscannerspiegel-Chip dem Betrag der Antriebsspannung direkt proportional und kann über diese geregelt werden. Durch das elektrostatische Antriebsprinzip sind Ablenkwinkel bis 60° erreichbar.

Prinzipiell sind für die Ansteuerung von Mikroscannerspiegeln elektrostatische, thermomechanische, piezoelektrische und magnetische Anregungsformen möglich. Aus den vielfältigen Möglichkeiten der Modulation bezüglich Frequenz, Amplitude und Intensität resultieren eine Vielzahl darstellbarer Abbildungen, wie beispielsweise Einfach- und Mehrfachspalte, Gitter sowie Raster.

Ein besonderer Vorteil der erfindungsgemäßen Beleuchtungseinheit besteht in der Möglichkeit einer quasi gleichzeitigen Abbildung von mehreren Spaltbildern auf oder in dem zu untersuchenden Auge. Dadurch kann die Dauer der Untersuchung verkürzt und die physische Belastung des Patienten wesentlich verringert werden.

Der so in seiner Ausbreitungsrichtung und durch zusätzliche Modulation auch in seiner Intensität veränderliche Laserstrahl **3** wird durch die Strahlformungs- und Strahllenkungselemente **5** auf oder in das zu untersuchende Auge **6** gelenkt. Die im Auge **6** entstehenden optischen Schnittbilder können in gewohnter Weise z. B. mit einem Stereomikroskop veränderbarer Vergrößerung oder einer ähnlichen Anordnung beobachtet werden. Heutige ophthalmologische Geräte besitzen neben einem Beobachtungssystem in der Regel auch eine Bildverarbeitungseinheit, mit deren Hilfe die Schnittbilder aufgenommen, weiterverarbeitet und ausgewertet werden können.

Die erfindungsgemäße Beleuchtungseinheit kann auf Grund ihrer geringen Abmessungen in verschiedenste ophthalmologische Untersuchungsgeräte, insbesondere Spaltlampen integriert werden. Die Beleuchtungseinheit kann dabei mit der herkömmlichen bereits vorhandenen Beleuchtungsanordnung kombiniert oder separat genutzt werden. Es ist ebenfalls möglich, die Beleuchtungseinheit als Zusatzmodul zur Erweiterung bereits vorhandener ophthalmologischer Geräte einzusetzen um dadurch die Untersuchungen am menschlichen Auge wesentlich vereinfachen und die Genauigkeit der Untersuchungsergebnisse verbessern zu können. Moderne ophthalmologische Untersuchungsgeräte verfügen über zusätzliche Dokumentationseinrichtungen wie beispielsweise Foto-/Videokomponenten zur analogen und digitalen Bildaufzeichnung und Bildbearbeitung und eine automatisierte Bildauswertung zur Gewinnung von Messwerten vom Untersuchungsobjekt. Zur Erhöhung der Schärfentiefe bei der Dokumentation kann die Aufnahmeeinheit mit ihrem Empfänger (z.B. CCD Chip) entsprechend der Scheimpflugbedingung abgestimmt auf den Winkel der Spalteinstrahlrichtung zur optischen Achse geneigt werden. Die Wirkung kann dabei vorteilhaft mit einem Monitorbild kontrolliert werden.

Bei der erfindungsgemäßen Beleuchtungseinheit kann die Intensität der Laserquelle vorteilhaft so gewählt werden, dass sie für die Beobachtung und Dokumentation ausreicht, der Durchmesser des Strahlenbündels jedoch fein genug für eine hohe Detailauflösung ist und Grenzwerte für die spektrale Bestrahlungsstärke am Augenhintergrund nicht überschritten werden. Durch eine geeignete Modulation der Intensität der Lichtquelle können weitere vorteilhafte Effekte, beispielsweise die räumliche Trennung von Projektionsstrukturen erreicht werden. Dazu verfügen die heutigen Laserdiodenmodule in der Regel über einen zusätzlichen Anschluß zum anlegen eines Modulationssignals. Auf diese Weise lassen sich verschiedene Beleuchtungsstrukturen wie Punkt- und Strichraster oder auch Mehrfachspaltabbildungen realisieren. Diese Beleuchtungsstrukturen können ebenfalls als dynamische Vorgänge erzeugt werden, um bestimmte Abläufe zu automatisieren.

Die beschriebene Beleuchtungseinheit dient der Erzeugung von optischen Schnittbildern nicht nur im Auge, sondern auch in anderen transparenten Medien. Die Beleuchtungseinheit ist beispielsweise auch zur Untersuchung von Flüssigkeitsschichten und/oder zur Prüfung von optischen Bauelementen, wie Linsen, Prismen usw. einsetzbar. Dabei kann es vorteilhaft sein, dass der Mikroscannerspiegel nicht in Schwingungen versetzt wird, sondern eine "langsame Scannbewegung" über das zu prüfende Objekt ausführt. Da nicht alle Mikroscannerspiegel die Möglichkeit für eine derartige Ansteuerung bieten, ist ein entsprechendes Modell auszuwählen.

## Patentansprüche

1. Beleuchtungseinheit zur Erzeugung von optischen Schnittbildern in transparenten Medien, insbesondere im Auge, bei der als Beleuchtungsquelle (1) ein Laser vorgesehen und zur gezielten Ablenkung der Laserstrahlen ein definiert steuerbares Reflexionselement (4) vorhanden sind, wobei das Reflexionselement (4) in Frequenz und Amplitude steuerbar und ein Spiegel geringer Abmessungen ist, **dadurch gekennzeichnet, dass** das Reflexionselement (4) in zwei oder mehreren Richtungen unabhängig voneinander kippbar ist.

2. Beleuchtungseinheit nach Anspruch 1, **dadurch gekennzeichnet, dass** die vom, als Beleuchtungsquelle (1) dienenden, Laser ausgesendete Strahlung eine geringe Divergenz aufweist und/oder dass die vom Laser ausgesendete Strahlung vorzugsweise im blauen oder grünen Spektralbereich liegt und/oder dass die Intensität der Beleuchtungseinheit (1) steuerbar ist und/oder dass die Beleuchtungseinheit (1) über Strahlformungselemente (2) verfügt.

3. Beleuchtungseinheit nach Anspruch 1 und 2, **dadurch gekennzeichnet, dass** die Beleuchtungsquelle (1) ein Laserdiodenmodul ist.

4. Beleuchtungseinheit nach Anspruch 1 bis 3, **dadurch gekennzeichnet, dass** das Reflexionselement (4) ein Mikroscannerspiegel-Chip beliebiger Bauart ist.

5. Ophthalmologisches Untersuchungsgerät, insbesondere Spaltlampe, **dadurch gekennzeichnet, dass** es eine Beleuchtungsanordnung nach Anspruch 1 bis 4 enthält, dass die Beleuchtungseinheit (1) kombiniert mit der herkömmlichen Beleuchtungseinrichtung genutzt wird und bei Bedarf zuschaltbar ist oder dass die Beleuchtungseinheit (1) separat zu der herkömmlichen Beleuchtungseinrichtung genutzt wird und optional ansetzbar ist, dass zusätzliche Dokumentationseinrichtungen wie beispielsweise Foto- oder Videokomponenten zur analogen und digitalen Bildaufzeichnung und Bildbearbeitung vorhanden sind, dass eine zusätzliche automatisierte Bildauswertung zur Gewinnung von Messwerten vom Untersuchungsobjekt vorhanden ist und dass die Dokumentationseinrichtung zur Erhöhung der Schärfentiefe gemäß der Scheimpflug-Bedingung um einen Winkel gekippt werden kann, um so die räumliche Ausdehnung des Schnittbildes in seiner gesamten Dimension besser erfassen zu können.

## Claims

1. Illumination unit for producing optical split images in transparent media, in particular in the eye, wherein a laser is provided as an illumination source (1) and a reflection element (4) which can be controlled in a defined manner is provided for directed deflection of the laser beams, wherein the reflection element (4) is controllable in frequency and amplitude and is a mirror of small dimensions, **characterised in that** the reflection element (4) can be tilted in two or more mutually independent directions.

2. Illumination unit as claimed in claim 1, **characterised in that** the beam emitted from the laser serving as an illumination source (1) has a low divergence and/or that the beam emitted from the laser preferably lies in the blue or green spectral range and/or that the intensity of the illumination unit (1) is controllable and/or that the illumination unit (1) has beam-forming elements (2).

3. Illumination unit as claimed in claim 1 and 2, **characterised in that** the illumination source (1) is a laser diode module.

4. Illumination unit as claimed in claim 1 to 3, **characterised in that** the reflection element (4) is a micro-scanner mirror chip of any construction type.

5. Ophthalmological examination device, in particular a slit lamp, **characterised in that** it contains an illumination arrangement as claimed in claim 1 to 4, that the illumination unit (1) is used in combination with the conventional illumination device and can be switched on if necessary or that the illumination unit (I) is used separately from the conventional illumination device and can optionally be added on, that additional documentation devices such as, e.g. photo or video components for analogue and digital image recording and image processing are provided, that an additional automated image evaluation for obtaining measurement values of the object under examination is provided and that the documentation device for increasing the depth of focus in accordance with the Scheimpflug condition can be tilted by an angle in order to be able better to detect the spatial expansion of the split image in its overall dimension.

## Revendications

1. Unité d'éclairage pour la production de vues en coupe optiques dans des milieux transparents, en particulier dans l'oeil, dans laquelle est prévu comme source d'éclairage (1) un laser, et pour la déviation ciblée des rayons laser, un élément de réflexion (4) pouvant être commandé d'une manière définie est prévu, où l'élément de réflexion (4) peut être commandé en fréquence et en amplitude et est un miroir de dimensions réduites, **caractérisée en ce que** l'élément de réflexion (4) peut être basculé dans deux ou plusieurs directions indépendamment les unes des autres.

2. Unité d'éclairage selon la revendication 1, **caractérisée en ce que** le rayonnement émis par le laser, servant de source d'éclairage (1), présente une faible divergence et/ou que le rayonnement émis par le laser se situe de préférence dans la zone spectrale bleue ou verte et/ou que l'intensité de l'unité d'éclairage (1) peut être commandée et/ou que l'unité d'éclairage (1) dispose d'éléments de formation de faisceau (2).

3. Unité d'éclairage selon la revendication 1 et 2, **caractérisée en ce que** la source d'éclairage (1) est un module à diode laser.

4. Unité d'éclairage selon la revendication 1 à 3, **caractérisée en ce que** l'élément de réflexion (4) est une puce de miroir de microscanner d'un type de construction sélectif.

5. Appareil d'examen ophtalmologique, en particulier lampe à fente, **caractérisé en ce qu'**il comporte un agencement d'éclairage selon les revendications 1 à 4, que l'unité d'éclairage (1) est utilisée en combinaison avec l'installation d'éclairage classique et peut être connectée en cas de besoin, ou bien que l'unité d'éclairage (1) est utilisée séparément de l'installation d'éclairage classique et peut être connectée en option, que des installations de documentation additionnelles comme par exemple des composants photo ou vidéo sont prévues pour l'enregistrement et le traitement analogique et numérique des images, **en ce qu'**il est prévu une évaluation d'images automatisée additionnelle pour obtenir des valeurs de mesure de l'objet d'examen, et **en ce que** l'installation de documentation, pour augmenter la profondeur de champ, peut être amenée, selon le principe de Scheimpflug, à basculer selon un angle pour pouvoir ainsi mieux détecter l'extension spatiale de la vue en coupe dans toute sa dimension.
